# EUROPEAN PATENT SPECIFICATION

(11) **EP 3 624 734 B1**
(45) Date of publication and mention of the grant of the patent: **18.08.2021**
(21) Application number: 18723846.4
(22) Date of filing: 16.05.2018
(51) Int. Cl.: A61F 2/28, A61C 8/02, A61C 8/00, A61B 17/80

(54) **FIXATION ELEMENT FOR OSTEOSYNTHESIS MEMBRANES**
FIXATIONSELEMENT FÜR OSTEOSYNTHESE-MEMBRANEN
ELEMENT DE FIXATION POUR MEMBRANES D'OSTEOSYNTHESE

(30) Priority: 16.05.2017 EP 17382280
(43) Date of publication of application: 25.03.2020
(73) Proprietor: Garcinuño Vazquez, Salvador, 07015 Palma de Mallorca (Illes Balears) (ES); Senís Segarra, Luís, 46002 Valencia (ES)
(72) Inventor: Garcinuño Vazquez, Salvador, 07015 Palma de Mallorca (Illes Balears) (ES); Senís Segarra, Luís, 46002 Valencia (ES)
(74) Representative: Pons Ariño, Angel
(86) International application number: PCT/EP2018/062695
(87) International publication number: WO 2018/210918

(56) References cited:
- US-A1- 2005 216 008
- US-A1- 2008 275 555
- US-A1- 2017 105 724
- US-B1- 6 187 009

## Description

### OBJECT OF THE INVENTION

The present invention lies within the technical field of devices especially adapted for osteosynthesis, as well as in the field of cortical plates for the jaws, and specifically refers to a fixation element for osteosynthesis membranes, especially designed to be arranged in the oral cavity. The closest prior art is document US 6187009 B1, which discloses a fixation element for osteosynthesis membranes comprising a pliable structure having a central plate of elongated geometry, two elongated lateral plates converging at the central plate, and a sharpened self-drilling point projected from a rear face of the structure to be driven into the bone structure.

### BACKGROUND OF THE INVENTION

Osteosynthesis is known as the surgical treatment of fractures in which the fractures are reduced and fixed by means of implanting artificial elements such as plates, screws, membranes or wires, said elements being made from biocompatible materials, such as surgical steel, titanium alloys or bioabsorbable polymers.

In the field of Maxillofacial Surgery, and more specifically in the speciality of Dentistry, the technique of Guided Bone Regeneration (GBR), in the area of osteosynthetic techniques, which is based on stimulating the growth of new bone in areas where there is bone loss, is known.

Said technique makes use of barriers or physical membranes in order to prevent gingival epithelial cells and connective tissue cells from invading the areas to be regenerated, thereby favoring the proliferation of osteoprogenitor cells for the growth of new bone.

In this technique, the membrane acts as a second flap and provides additional protection to the wound, reducing impact to the wound and guaranteeing the differentiation of the mesenchymal cells into osteoblasts instead of fibroblasts.

GBR therefore implies the placement of a barrier membrane which covers the defective bone, separating it from the gingival tissue and preventing the direct contact thereof with the bone during the healing process. This allows for bone regeneration and for the bone defect to be filled.

Clinical and histological studies have shown that osteosynthetic barrier membranes must be perfectly adapted to the peripheral bone, that is, to the defect, forming a seal which prevents gingival connective tissue cells from invading the space formed below the membrane, since these cells compete with the bone-forming cells. Therefore, it is essential that the membrane be stable during the healing period.

Likewise, among the different types of membranes, both resorbable and non-resorbable membranes are known. Resorbable membranes, of either natural or synthetic origin, have the disadvantage of a loss of stability over time, and even on occasions they may cause inflammation associated with the degradation of the membrane and the loss of rigidity.

Non-resorbable membranes have great structural integrity, and therefore they maintain the essential characteristics thereof over time. They normally provide better results than resorbable membranes, since its barrier effect is more prolonged in time. The only drawback in the use of said membranes is that they must be removed by means of a second surgery.

In order to be correctly secured to the bone, the membranes require auxiliary fixation elements, which provide the necessary rigidity to said membrane while allowing for a pivoting movement, if necessary, to facilitate the adaptation of the membrane to the geometry of the specific anatomical region in which they are placed, as well as the changes produced over time due to bone growth. Likewise, in the specific case of non-resorbable membranes, said fixation elements must be easily extractable.

US patent US2014248583 discloses an apparatus and a method for supporting an intraosseous implant that facilitates and prevents further resorption at an implantation site by reducing parafunctional pressures experienced at the implantation site, transferring and transmitting functional pressures to the implantation site without an increase in size of an intraosseous implant and without osteosynthesis.

Due to its morphology, said apparatus would not be useful for the maxillary sinus in the event of a hypothetical gash of Schneiderian membrane caused by an elevation of the own sinus.

### DESCRIPTION OF THE INVENTION

The invention is defined in claim 1. The object of the invention consists of a fixation element for osteosynthetic barrier membranes, specially designed for those which are intended to be inserted inside the oral cavity, which allows for the binding of said membranes to a bone in order to favor the proliferation of osteoprogenitor cells in an area where a bone defect has been produced, so that said cells cause new bone growth.

Additionally, the fixation element described in the present invention may also be applied in cases of peri-implantitis, with exposure of the dental implant due to bone loss, in which the fixation element acts as a lateral barrier, thereby avoiding shearing movements which may cause loss of the implant.

In cases of implantation with deficient primary stability of the implant, as well as in the immediate post-extraction placement of dental implants, the fixation element secures said implant, providing it with the necessary stability to favor the process of osteointegration. Lastly, the fixation element also ensures the fixation of any bone filling around the exposed part of the implant.

To do so, the fixation element to which the present invention relates consists of a laminated pliable structure formed by three plates, namely two lateral plates and a central plate, connected to each other by one of the respective ends thereof, thereby forming an angle between them, which is preferably a 45° angle, with a resulting geometry similar to an arrow or eagle's claw. The laminated structure is made of materials that are pliable and biocompatible with the human body, such as stainless steels, chrome-cobalt alloys, titanium, titanium alloys, nickel, titanium-nickel alloys, gold, gold alloys, tantalum, methacrylate, polyethylene, or ceramics.

In a preferred embodiment, the laminated structure is made from grade 2 type 4 titanium, with a richness in Ti of at least 99,3%, being the other elements of the alloy Fe and O.

The lateral plates, with an essentially elongated geometry, converge at a fixation point, forming an angle between them, preferably 90°. From said fixation point, the central plate extends, also with an essentially elongated geometry, such that a central axis of said central plate is the bisecting line of the angle formed by the lateral plates. Thus, the angle formed between the central plate and each one of the lateral plates in the preferred embodiment is 45°, consisting of the aforementioned geometry. Said lateral plates, one or both of them simultaneously, can also show an internally oriented curvature, therefore forming a plate parallel to the central plate on its distal end, similarly to a fork or trident.

The element also incorporates fixation means to facilitate the anchoring thereof to the bone, said anchoring being temporary in the case that the osteosynthetic membrane is non-resorbable.

In a preferred embodiment, said fixation means consist of self-drilling sharpened points which are projected toward one of the faces of the laminated structure, preferably intended to bind to the bone of the oral cavity by being imbedded therein. Said self-drilling points are preferably located on the fixation point of the three plates, as well as on the free ends of each of the lateral and central plates.

Said free ends from which self-drilling points are projected further incorporate distal bulges, preferably round-shaped, intended to allow a homogeneous force distribution in the event of impacts exerted on the point in order to fix them. Likewise, said distal bulges create a supplementary are on which exert a lever force in order to retire the fixation element.

The point located on the fixation point of the three plates enables a pivoting motion of the element with respect to the bone, thereby facilitating the adaptation thereof, and that of the membrane, to the geometry of the anatomical area on which it is intended to be placed. Furthermore, this area changes over time, due to the growth of the bone. In turn, the points located on the free ends of the plates are preferably used on jaws and cortical bones to obtain a supplementary fixation of the element. In case said points are not needed, they can be easily removed from the laminated structure by cutting.

Self-drilling points are part of the laminated structure and are therefore made on the same material, it is to say, preferably titanium. This gives said points a hardness enough to penetrate on bone tissues, even just pressing the without impacts.

Likewise, the option of a laminated structure incorporates on at least one of the plates of the laminar structure a central bulge, which defines, if necessary, a through perforation which allows for the passage therethrough of additional fixation elements to the bone, such as suture thread and the corresponding needle, to thereby ensure the stability and success of the graft. Said central bulge is preferably round-shaped, as above-mentioned distal bulges.

Said through perforation can be made, if necessary, in any of the distal bulges located on the free ends, after cutting corresponding self-drilling point. Preferably the perforation is sized to allow the passage therethrough of only the suture thread and its corresponding needle, therefore said perforation is adapted to the maximum diameter that allows the passage therethrough of of the suture thread and its corresponding needle. The skilled in the art knows the diameters of suture threads used with the purposes of this invention. Said perforation is defined so that it does not allow the passage therethrough of any kind of the screws used on the fixation of structures to the bone.

Due to the reduced thickness and the pliability of the laminated structure, the mentioned perforation may be done manually by means of pressure with a sharp element, such as scissors. Likewise, if necessary, said features allow the simple elimination by means of cutting with surgical scissors, of part of the plates, to better adapt to the morphology of the defect on which it is placed.

In specific cases in which the fixation element is arranged on a trabecular bone or on maxillary bones, the incorporation of additional fixation elements to the bone is envisaged. Preferably, said additional fixation elements consist of conical pins comprising a plug body formed from a sequence of four truncated cones defining a toothed profile which allows the resulting pin to couple to the bone tissue with the ability to retain the same.

The portion of the head of the pin is used to couple to the fixation element with the corresponding membrane used in osteosynthesis and to hold it in the proper position on the structure of the underlying tissue. The pin may be made of a resorbable polymer, such as polydioxanone (PDS) and has a substantially sold construction in all parts thereof.

In an alternative embodiment, a circularly cylindrical extension is provided between the larger truncated cone and the head of the pin. This arrangement maintains a separation between the barrier membrane and the underlying tissue.

In a preferred embodiment, the fixation element is made in a single compact piece by any suitable proceeding thereof. Manufacturing can be performed, for instance, by die-cast, inlaying or 3D printing. Said single piece allows the performance of direct fixations by pressing, which is advantageous in some anatomical areas, as well as it avoids the need to make additional perforations. The compact piece also results in a continuous surface which can be completely sterilized, and it allows its fixation by a surgeon with just one hand.

The fixation element of the osteosynthesis membranes described herein is easily attachable and removable, and provides a secure and stable fixation of the osteosynthetic membranes over time, allowing for their adaptation to a variable anatomy over time. It is envisaged that said fixation element forms part of a fixation kit of osteosynthesis membranes, also comprised of a hammer, and impactor and an extractor.

The fixation element according to the invention is configured for use in maxillary sinus elevation procedures. Said procedures start with the performance of a lateral window therethrough the Schneiderian membrane is deployed and separated and, afterwards said sinus is replenished with any kind of osteosynthetic biomaterial, therefore regenerating the area with a new bone that allows the fixation of an implant.

An eventual break of the Schneiderian membrane, which is very fragile due to its reduced thickness, would cause the introduction of granules of the osteosynthetic biomaterial into the sinus, therefore causing a sinusitis. A solution would consist of inserting an artificial membrane which would block the passage of said granules, although said artificial membrane would not maintain its optimal position without the addition of a fixation element with the cavity performed into the sinus.

The fixation element as described before allows, thanks to the combination of its arrowhead geometry and the presence of the self-drilling points, the fixation of an and of the artificial membrane to the bone from the outer part of the cavity. Likewise, the pliability of its three plates allows an easy fastening, therefore blocking the passage of any undesired element into the sinus.

Once said passage is blocked, the cavity can be replenished with the selected osteosynthetic biomaterial. Afterwards, the window is closed with another membrane, preferably made of collagen, to avoid the exit of the biomaterial, and the wound is closed with suture threads.

On the other hand, thanks to its pliability and its self-drilling character, the fixation element also allows the performance of provisional fixation of flaps of skin, fixing said soft tissues to the palate or other areas of oral cavity, therefore allowing a better performance of the procedure. Once said performance is made, the fixation element is retired and the flaps of skin can be sewed up, therefore allowing the closure of the wound with corresponding suture threads.

### DESCRIPTION OF THE DRAWINGS

As a complement to the present description, and for the purpose of helping to make the characteristics of the invention more readily understandable, in accordance with a preferred practical exemplary embodiment thereof, said description is accompanied by a set of drawings constituting an integral part of the same, which by way of illustration and not limitation represent the following:

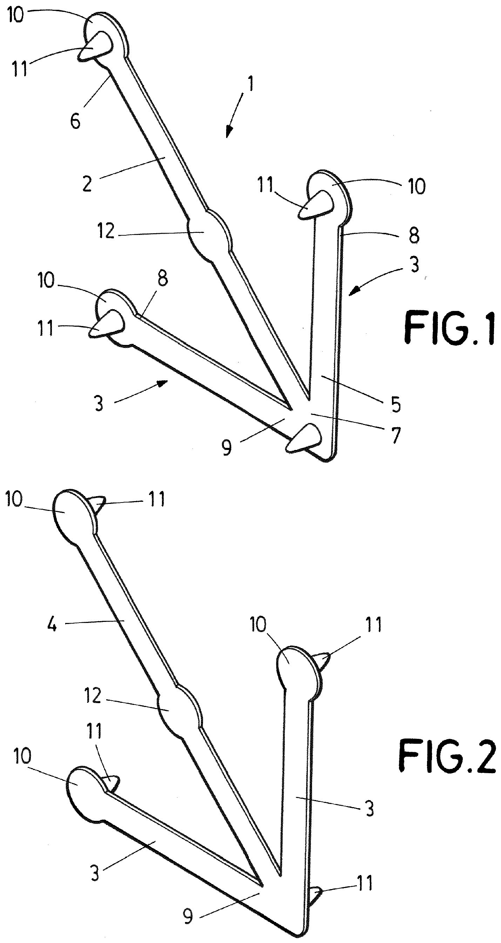

Figure 1 shows a front perspective view of the fixation element, in which the main constituting elements thereof can be seen.
Figure 2 shows a rear perspective view of the element illustrated in figure 1.
Figure 3 shows a front perspective view of a detail of the fixation element according to the invention.
Figure 4 shows a front perspective view of the fixation element according to the invention, used in a maxillary sinus elevation procedure.

### PREFERRED EMBODIMENT OF THE INVENTION

What follows is a detailed description, with the help of the figures referenced above, of an exemplary fixation element.

The fixation element for osteosynthesis membranes described herein is made up of a pliable laminated structure (1) of a similar geometry to that of an arrowhead as shown in the attached figures, laminated structure (1) which is made from grade 2 type 4 titanium, with a richness in Ti of at least 99,3%, This laminated structure (1) in turn, comprises a central plate (2) and two lateral plates (3), converging at a central plate (2).

The laminated structure (1) has a front face (4), intended to be oriented towards the outside and a rear face (5), opposite the front face (4), intended to be facing a bone structure of an individual in direct contact with a membrane of the osteosynthesis, not shown in the attached figures.

The central plate (2), of an elongated geometry, has a first free end (6) and a first fixation end (7). In turn, each one of the lateral plates (3), also of an essentially elongated geometry, has a second free end (8) and a second fixation end (9), which converges at the first fixation end (7) of the central plate (2).

Distal bulges (10) of an essentially round shape are located on the first free end (6) as well as on both second free ends (8). Respective sharpened self-drilling points (11) are projected from each of said distal bulges (10), as well as from the convergence of the fixation ends (7, 9), from the rear face (5) of the laminated structure (1).

Self-drilling points (11) can be driven into the bone structure and allow for a quick and easy removal thereof if necessary, as well as they allow for pivoting motion of the laminated structure (1) in order to facilitate the adaption of the fixation element, and of the membrane of the osteosynthesis, to the anatomy of the area in which it is intended to be connected. Moreover, it is necessary to consider the fact that the geometry and dimensions of said anatomy is variable over time, due to the bone growth and development produced.

The central plate (2) has a central bulge (12) which has an essentially circular geometry, located on the central segment of said central plate (2).

Defined on said central bulge (12), if necessary, is a small through perforation, not shown in the attached figures, to allow for the passage therethrough of a suture thread and the corresponding needle, to thereby ensure the stability and success of the graft.

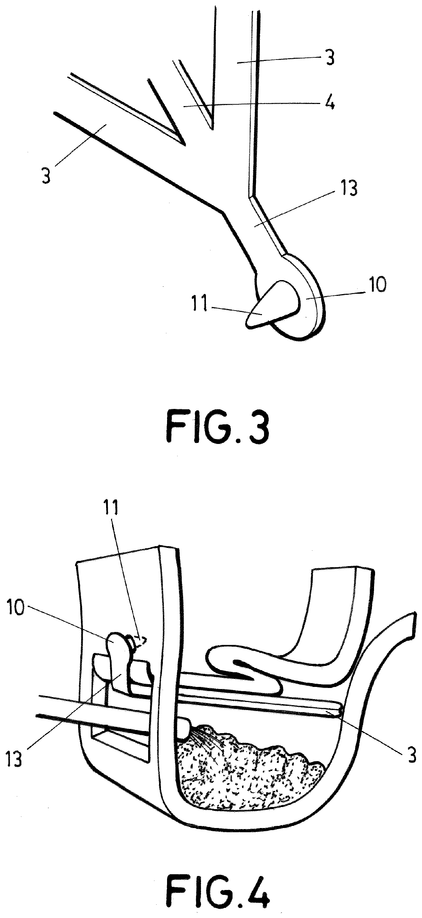

Figure 3 shows an embodiment of the invention, in which an extension (13) is projected from the convergence of the fixation ends (7, 9), on the same direction as the central plate (2), extension (13) that comprises a free end on which another distal bulge (10), with a corresponding self-drilling point (11), is located.

Figure 4 shows this preferred embodiment used in a maxillary sinus elevation procedure, on which it can be seen that said extension (13) allows the fixation to a maxillary bone.

## Claims

1. A fixation element for osteosynthesis membranes, bindable to a bone structure, the fixation element comprising a pliable laminated structure (1) of an arrowhead geometry
which has:
- a front face (4), intended to be oriented towards the outside, and
- a rear face (5), opposite the front face (4), intended to face and be in direct contact with a membrane of the osteosynthesis which, in turn, is connected to the bone structure,
wherein the laminated structure (1) in turn comprises:
- a central plate (2) of an elongated geometry, which has a first free end (6), and a first fixation end (7), and
- two lateral plates (3), converging at the central plate (2), each one of which has a second free end (8), and a second fixation end (9), which converges at the first fixation end (7) of the central plate (2),
wherein the fixation element further comprises distal bulges (10) located on the first free end (6) and on each of the second free ends (8), and the pliable laminated structure (1) further incorporates an extension (13) that is projected from the convergence of the fixation ends (7, 9), the extension (13) comprising a free end on which another distal bulge (10) is located, wherein a sharpened self-drilling point (11) is projected from the distal bulge at the free end of the extension from the rear face of the laminated structure (1) to be driven into the bone structure, whereby the fixation element is configured for use in the prevention of sinusitis after a maxillary sinus elevation procedure.

2. The fixation element for osteosynthesis membranes according to claim 1, wherein the fixation plate further comprises additional self-drilling points (11) which are projected from each of the distal bulges (10), from the rear face (5) of the laminated structure (1).

3. The fixation element for osteosynthesis membranes according to any of the preceding claims, wherein the central plate (2) incorporates a central bulge (12).

4. The fixation element for osteosynthesis membranes according to claim 1, wherein the extension (13) is projected in the same direction as the central plate (2).

5. The fixation element for osteosynthesis membranes according to any of the preceding claims, wherein the pliable laminated structure (1) is made from grade 2 type 4 titanium.

## Patentansprüche

1. Befestigungselement für Osteosynthesemembranen, das an eine Knochenstruktur bindefähig ist, wobei das Befestigungselement eine nachgiebige laminierte Struktur (1) einer Pfeilspitzengeometrie umfasst, die Folgendes aufweist:
- eine Vorderseite (4), die nach außen gerichtet sein soll, und
- eine der Vorderseite (4) gegenüberliegende Rückseite (5), die einer Membran der Osteosynthese, die ihrerseits mit der Knochenstruktur verbunden ist, zugewandt sein und in direktem Kontakt mit dieser stehen soll,
wobei die laminierte Struktur (1) wiederum umfasst:
- eine Mittelplatte (2) mit länglicher Geometrie, die ein erstes freies Ende (6) und ein erstes Befestigungsende (7) aufweist, und
- zwei seitliche Platten (3), die an der Mittelplatte (2) konvergieren, von denen jede ein zweites freies Ende (8) aufweist, und ein zweites Befestigungsende (9), das am ersten Befestigungsende (7) der Mittelplatte (2) konvergiert,
wobei das Befestigungselement ferner umfasst:
- distale Ausbuchtungen (10), die sich am ersten freien Ende (6) und an jedem der zweiten freien Enden (8) befinden, und wobei die biegsame laminierte Struktur (1) ferner eine Verlängerung (13) integriert, die von der Konvergenz der Befestigungsenden (7, 9) hervorsteht, wobei die Verlängerung (13) ein freies Ende umfasst, an dem sich eine weitere distale Ausbuchtung (10) befindet, wobei eine geschärfte selbstbohrende Spitze (11) von der distalen Ausbuchtung am freien Ende der Verlängerung von der Rückseite der laminierten Struktur (1) hervorsteht, um in die Knochenstruktur eingetrieben zu werden, wodurch das Befestigungselement zur Verwendung bei der Vorbeugung von Sinusitis nach einer Kieferhöhlen-Elevationsprozedur konfiguriert ist.

2. Befestigungselement für Osteosynthesemembranen nach Anspruch 1, wobei die Befestigungsplatte ferner zusätzliche selbstbohrende Spitzen (11) umfasst, die von jeder der distalen Ausbuchtungen (10) von der Rückseite (5) der laminierten Struktur (1) hervorstehen.

3. Befestigungselement für Osteosynthesemembranen nach einem der vorhergehenden Ansprüche, wobei die Mittelplatte (2) eine mittlere Ausbuchtung (12) integriert.

4. Befestigungselement für Osteosynthesemembranen nach Anspruch 1, wobei die Verlängerung (13) in die gleiche Richtung wie die Mittelplatte (2) hervorsteht.

5. Befestigungselement für Osteosynthesemembranen nach einem der vorhergehenden Ansprüche, wobei die biegsame laminierte Struktur (1) aus Titan Grad 2 Typ 4 hergestellt ist.

## Revendications

1. Élément de fixation pour des membranes d'ostéosynthèse, pouvant être lié à une structure osseuse, l'élément de fixation comprenant une structure stratifiée pliable (1) de géométrie en forme de pointe de flèche qui a :
- une face avant (4), destinée à être orientée vers l'extérieur, et
- une face arrière (5), à l'opposé de la face avant (4), destinée à faire face et à être en contact direct avec une membrane de l'ostéosynthèse qui, à son tour, est reliée à la structure osseuse,
dans lequel la structure stratifiée (1) comprend à son tour :
- une plaque centrale (2) de géométrie allongée, qui a une première extrémité libre (6), et une première extrémité de fixation (7), et
- deux plaques latérales (3), convergeant vers la plaque centrale (2), dont chacune a une seconde extrémité libre (8), et une seconde extrémité de fixation (9), qui converge vers la première extrémité de fixation (7) de la plaque centrale (2),
dans lequel l'élément de fixation comprend en outre
- des renflements distaux (10) situés sur la première extrémité libre (6) et sur chacune des secondes extrémités libres (8), et la structure stratifiée pliable (1) intègre en outre une extension (13) qui fait saillie depuis la convergence des extrémités de fixation (7, 9), l'extension (13) comprenant une extrémité libre sur laquelle un autre renflement distal (10) est situé, dans lequel une pointe autoperceuse aiguisée (11) fait saillie depuis le renflement distal à l'extrémité libre de l'extension depuis la surface arrière de la structure stratifiée (1) pour être enfoncée dans la structure osseuse, selon lequel l'élément de fixation est configuré pour être utilisé dans la prévention de la sinusite après une procédure d'élévation du sinus maxillaire.

2. Élément de fixation pour des membranes d'ostéosynthèse selon la revendication 1, dans lequel la plaque de fixation comprend en outre des pointes autoperceuses supplémentaires (11) qui font saillie depuis chacun des renflements distaux (10), depuis la face arrière (5) de la structure stratifiée (1).

3. Élément de fixation pour des membranes d'ostéosynthèse selon l'une quelconque des revendications précédentes, dans lequel la plaque centrale (2) intègre un renflement central (12).

4. Élément de fixation pour des membranes d'ostéosynthèse selon la revendication 1, dans lequel l'extension (13) fait saillie dans la même direction que la plaque centrale (2).

5. Élément de fixation pour des membranes d'ostéosynthèse selon l'une quelconque des revendications précédentes, dans lequel la structure stratifiée pliable (1 ) est fabriquée à partir de titane de type 4 grade 2.
